# EUROPEAN PATENT SPECIFICATION

(11) **EP 2 628 445 B1**
(45) Date of publication and mention of the grant of the patent: **30.01.2019**
(21) Application number: 12155867.0
(22) Date of filing: 17.02.2012
(51) Int. Cl.: A61B 5/097, A61M 16/00

(54) **APPARATUS FOR ANALYZING BREATHING GAS FLOWING ALONG A BREATHING TUBING FOR SUBJECT BREATHING**
VORRICHTUNG ZUR ANALYSE VON ENTLANG EINES BEATMUNGSSCHLAUCHS STRÖMENDEM ATEMGAS ZUR BEATMUNG EINES PATIENTEN
APPAREIL POUR L'ANALYSE DE GAZ RESPIRATOIRE CIRCULANT LE LONG D'UN TUBAGE RESPIRATOIRE POUR LA RESPIRATION D'UN SUJET

(43) Date of publication of application: 21.08.2013
(73) Proprietor: General Electric Company, Schenectady, NY 12345 (US)
(72) Inventor: HAVERI, Heikki Antti Mikael, 03150 Huhmar (FI)
(74) Representative: Illingworth-Law, William Illingworth

(56) References cited:
- US-A- 5 322 057
- US-A1- 2005 284 469
- US-A1- 2006 086 254
- US-A1- 2011 000 488
- US-A1- 2011 265 793

## Description

### BACKGROUND OF THE INVENTION

This disclosure relates generally to an apparatus and method for analyzing a breathing gas flowing along a breathing tubing for subject breathing. Liquid particles are delivered at times depending on a phase of the breathing cycle or continuously into the breathing gas. Also this disclosure relates to a corresponding arrangement.

A tidal volume (TV) is an amount of an air inspired or taken into lungs in a single breath. TV is dependent on the sex, size, height, age and a health etc. of a patient, but in general TV also decreases as the size of the patient decreases. In an average healthy adult, TV is about 400-600 ml whereas in an average healthy neonate, that measures 3.5-4 kg and is 50 cm tall, TV is approximately 25-50 ml. On the other hand, in an average premature neonate that measures only 500 grams TV is only about 2-3.5 ml. TV of a smaller patient's is very difficult to measure, but it can be approximated to 4-7 ml/kg, applying a general rule of thumb for approximating the TV of the human lung. In practice the TV of the patient suffering pulmonary system deficiency is normally much less than the approximation gives.

A respiration rate (RR) is dependent on the sex, size, height, age and a health etc. of the patient, but in general RR increases as the size of the patient decreases. In an average healthy adult, RR is about 10-20 breaths/minute, whereas RR of a neonate may exceed as high as 150 breaths/minute.

When the patient is mechanically ventilated with a conventional ventilator, an endotracheal tube is placed into a trachea so that it goes through oral or nasal cavity and larynx. The other end of the endotracheal tube is connected to a breathing circuit Y-piece through a luer type connector. If the patient is gas monitored with a mainstream or sidestream gas analyzer, an airway adapter used for sampling the breathing gas that is analyzed by the gas analyzer is normally connected between the endotracheal tube and the breathing circuit Y-piece connectors. During an inspiration the fresh breathing gas including higher oxygen (O₂) concentration flows into the patients' lungs through an inspiratory limb of the breathing circuit Y-piece, the airway adapter, the endotracheal tube and their connectors, then to a trachea, a bronchus, a bronchi, bronchioles and finally reaching an alveoli deep in the lungs, where all the gas exchange actually occurs. Carbon dioxide (CO₂) molecules in a hemoglobin of a blood flowing in tiny blood vessels around the alveoli are replaced with O₂ molecules in the fresh breathing gas through the thin walls of the alveoli. O₂ molecules take their place in the hemoglobin, whereas CO₂ molecules flow out from the patient within the used expired breathing gas, through the same path as the fresh gas came in during the inspiration. Thus a gas concentration of the breathing gas measured by the gas analyzer is somewhat proportional to the gas concentration in the blood.

A volume in a space between a intersection of the inspiratory and expiratory limbs of the Y-piece and the patient's mouth or nose, a beginning of oral and nasal cavities, is called a mechanical dead volume or dead space, whereas the volume in a space between patient's mouth or nose and the entrance of alveoli is called an anatomical dead volume. The part of the lung that is injured or damaged for some reason and does not participate for the gas exchange is called more specific a physical dead volume. It is obvious that as the used breathing gas flows out from the patient's lungs through the expiratory limb during expiration, a part of the used gas newer exits a pulmonary system, as well as the patient side of the breathing circuit, but remains in the mechanical and anatomical dead volume. Then as the fresh gas is inspired in to the lungs through the inspiratory limb the used gas already in the anatomical and mechanical dead volume flows into the lungs before the fresh gas. The used gas fills up some or all of the alveoli depending on a ratio of the dead volume and TV or at least mixes up with the fresh gas decreasing the concentration of O₂ as well as increasing the concentration of CO₂ in the lungs, which in turn decreases the gas exchange in the alveoli. This means that the larger the dead space, the larger the volume of the used gas, with a low O₂ and high CO₂ concentration, that flows back to the patients' lungs during the inspiration and worse the gas exchange in the alveoli. In other words, if the total dead volume were larger than TV or as large as TV, the patient would not get any fresh gas into the lungs, but respires the used gas back and forth in the dead volume. In practice a diffusion of gases assists the gas exchange over the dead volume little, especially when there is some movement of gases such as a high frequency ventilation evolved, but the overall gas exchange in the alveoli would be lethal or dangerously poor anyway.

The anatomical dead volume is almost impossible to reduce, but it is proportional to the size and the physical condition of the patient. The mechanical dead volume depends on a breathing circuit design, an inner diameter of breathing circuit tubing, connectors and additional accessories, such as airway adapters used with a sidestream and mainstream gas analyzers. Obviously it is optimal that the mechanical dead space is zero as with normal breathing. It is also obvious that the mechanical dead volume is more critical for smaller patients with smaller TV or patients suffering barotraumas etc., which decrease TV.

The mainstream gas analyzing is suitable for intubated patients or patients wearing face mask or nasal mask in general. Mainstream analyzers are placed between the breathing circuit Y-piece and endotracheal tube through their airway accessory used for measuring the gas concentration of the gas flowing through the analyzer. However, existing mainstream gas analyzers are big and heavy and thus very impractical to use especially with small patients as they cover the patients face and tiny endotracheal tube easily bents and clock under the weight. Furthermore accessories and additional connectors add the dead space considerably, which is critical for a small patient with small TV. Also the design of airway adapters and their non-tubular gas sampling chambers and connectors are inefficient and they generate turbulences in to the breathing gas flow mixing end tidal gas with fresh gas columns, thus mixing the gas samples that the mainstream analyzer tries to analyze, causing measurement inaccuracy especially with higher RR and small TV. In practice existing mainstream analyzers are not used with smaller patients at the moment at all.

The sidestream gas analyzing can be used with intubated and non-intubated patients. Sidestream analyzers are usually big and heavy and comprise complicated gas analyzing technology, thus they are placed further away from a patient for example inside the host device such as patient monitor or ventilator. Gas samples are sucked actively into the analyzer with a gas pump through a sampling tube. When intubated patients are measured, the sampling tube is connected to a port in airway adapter, which is placed between the breathing circuit Y-piece and endotracheal tube. Gas samples are sucked through the port, which is in connection with the breathing gas flowing through the airway adapter. When measuring non-intubated patients the sampling tube can be connected to nasal prongs, masks or straight for example into the nasal cavity to take gas samples from the gas flowing through patient's upper airways.

The distance between the patient and the analyzer is usually very long between 2-6 meters, which means that gas samples travel a long distance before entering the analyzer. Gas samples sucked from the patient's breathing gas flow through the port in the airway adapter, through a connector between sampling tube and the port, then through 2-6 m long tiny tubing, which inner diameter is usually between 0.8-1.5 mm, through filters that separate water, mucus, blood etc. and finally through membrane tubing having ionic properties that transfers water molecules trough the membrane to even the humidity between the sample gas and the ambience. It is also possible to place filters between the port in airway adapter and sampling tube followed by the membrane tubing to prevent liquids to enter sampling tubing.

As gas samples, or columns of gas including mixture of gases with different concentrations, travel through connectors, sample tubing and filters, they mix up and average along the long path degrading the gas concentration measurement accuracy considerably. Furthermore, the measurement accuracy degrades rapidly, especially when RR increases and TV degreases. This can be seen as damped and rounded capnogram, which is due to increased number of smaller samples or shortened gas columns that mix up and average easier causing the amplitude of measured gas to decrease rapidly. The flow rate of sample gas has an effect on gas sample averaging. The lower the sample gas flow, the longer the gas columns travel through the tubing etc. and the more they mix up and average. Sample gas flow rates of existing sidestream gas analyzers are usually between 50-400 ml/min. As the flow rate of the sample gas is decreased for example from 200 ml/min to 50 ml/min the sensitivity to breathing gas concentration changes decreases as the sample gas travels longer inside the tubing etc. and mixes up and averages more. It is possible to increase the flow rate of sample gas through the tube by decreasing the inner diameter of tube. However, the negative pressure enabling the sample gas flow must be increased to keep the flow speed equal. Higher negative pressure means more power full pump. Gas columns may average even more as the flow speed of gas at the inner surface of tube is zero and maximum in the middle of the tube. Smaller diameter tubing also tends to clog easier. For these reasons most of the sidestream gas analyzer manufacturers specify the measurement range for RR only, which may go up frequencies of 120-150 breaths/minute, but the accuracy of the gas concentration measurement is not specified or it is specified up to 15-60 breaths/minute only, which is usable for adults only.

Nebulizer is a device used to deliver liquid form drugs into the patient's lungs in the form of mist of small droplets called aerosol. Existing nebulizers are usually very big, clumsy, position sensitive and they produce the aerosol continuously. Nebulizers are commonly placed between the inspiratory line, between the inspiratory limb of the breathing circuit Y-piece and the ventilator. Sometimes nebulizers are connected between the endotracheal tube and a manual resuscitator, especially when smaller patients are treated, but then the patient is naturally disconnected from the mechanical ventilator and ventilated manually. The aim is to produce the aerosol into the flowing inspiratory air to enable the aerosolized drug to enter the patient's lungs. However, as the existing nebulizers produce the aerosol continuously, during both inspiration and expiration and as the I:E ratio of ventilation may be 1:1 or preferably 1:2, only a small part of the drug really flow towards the patient's lungs.

When the nebulizer is connected between the inspiratory line, almost the whole line is filled up with aerosol continuously. During inspiration only the gas column in the inspiratory line close to the Y-piece, which volume is proportional to patient's TV, flows towards the patient filling up the mechanical dead volume as well. When expiration starts, the aerosol in the dead volume flows towards the ventilator, but as the nebulizer produces the aerosol continuously, the aerosol produced during expiration flows straight out from the inspiratory line through the Y-piece into the expiratory line as well. If existing nebulizers are placed between the breathing circuit Y-piece and the endotracheal tube, part of the aerosol produced during inspiration flow towards the patient, but aerosol generated during the expiration flow within the expired air away from the patient into the ventilator and other devices connected to breathing circuit. Another general problem with continuous aerosol production is similar to over humidification where droplets produced into the motionless air hit each other combining into larger droplets and they also hit the breathing circuit walls finally turning into liquid. Liquid then floats back and forth in the breathing circuit with the flowing air and may enter the patient's airways causing temporary airway obstruction or even drowning or liquid may enter sensitive devices and analyzers connected to breathing circuit causing malfunction or even breakdown. There has been attempts for nebulization that can be turned on and off in regard to ventilation to increase the delivery efficiency, but there does not exist functioning devices on the markets at the moment.

The functioning of other devices, such as for example sidestream gas analyzers that are connected between the breathing circuit Y-piece and the endotracheal tube, suffer from the aerosol or liquefied aerosol flowing back and forth within the flowing air. The optimum mean droplet diameter to ensure aerosol delivery into the deep lung and alveoli is between 1 and 5 microns thus this size droplets easily enter the smallest cavities of devices connected to breathing circuit as well. It is obvious that aerosol flowing through the airway adapter disturb the sidestream gas analyzers especially during inspiration as they continuously suck gas samples and aerosol within the gas sample from the breathing circuit to generate a continuous real time capnogram of inspiratory and expiratory gases. Aerosol particles and liquefied aerosol easily flow into the sampling tubing clogging the sampling tube and may even enter the filters and membrane tubing clogging them as well. Many aerosolized drugs may include alcohol or other chemicals that may disturb and even destroy the functioning of filters and membranes when the aerosol or liquid may enter the very sensitive analyzing components destroying the whole analyzer.

Thus, the existing sidestream gas analyzers are not very functional with existing nebulization as described earlier. Gas analyzing needs to be stopped and/or removed from the breathing circuit for the time of nebulization to prevent device malfunction. Gas analyzing would also generate false measurement values during the nebulization that would further lead to incorrect estimation of patient's condition and care practice.

US 2011/0265793 discloses a method and device for maintaining a volume of a breathing gas in a desired level when ventilating a subject. A gas sample is withdrawn from the volume of the breathing gas for an analysis decreasing the volume of the breathing gas. The volume of the gas sample is determined and based on this determined volume a volume of compensation gas is delivered into the breathing gas for maintaining the breathing gas volume in the desired level.
US 2005/284469 A1 discloses integrating a ventilator and a nebulizer. US 5 322 057 A discloses a nebulizer for use in conjunction with mechanical respirators.

### BRIEF DESCRIPTION OF THE INVENTION

The above-mentioned shortcomings, disadvantages and problems are addressed herein which will be understood by reading and understanding the following specification.
The present invention is defined in the accompanying claims.

In an example, an apparatus for analyzing a breathing gas flowing along a breathing tubing for subject breathing, the breathing gas comprising breathing cycles having different phases, liquid particles being delivered at times depending on the phase of the breathing cycle or continuously into the breathing gas is disclosed. The apparatus includes a gas sample supplier to adjust gas sample supply from the breathing gas and a gas analyzer to receive the gas sample adjusted by the gas sample supplier and to measure the gas sample property of the breathing gas. The apparatus further includes a processing unit to receive a signal indicative of one of the phase of the breathing cycle and the delivery timing of the liquid particles. The processing unit is adapted to control the gas sample supplier based on the signal received by the processing unit to limit access of liquid particles with the gas sample towards the gas analyzer.

Various other features, objects, and advantages of the invention will be made apparent to those skilled in art from the accompanying drawings and detailed description thereof.

### BRIEF DESCRIPTION OF THE DRAWINGS

Figure 1 is a schematic view of an arrangement in accordance with an example; and
Figure 2 is a detailed schematic view of a part of the arrangement in Figure 1 including also an apparatus.

### DETAILED DESCRIPTION OF THE INVENTION

Specific examples are explained in the following detailed description making a reference to accompanying drawings. These detailed examples can naturally be modified and should not limit the scope of the invention as set forth in the claims.

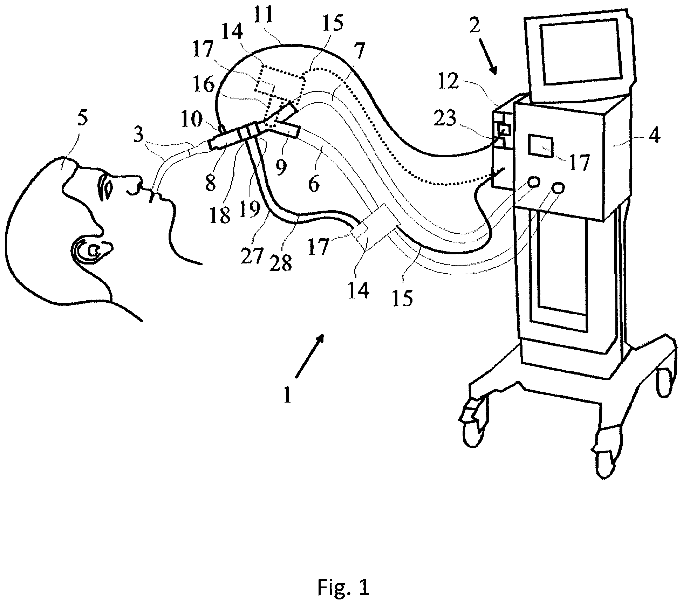

To enable the gas analyzing during liquid supply such as nebulization or humidification the effects of liquid particles such as aerosolized liquids to gas analyzing as described earlier needs to be minimized or eliminated. Figure 1 shows a schematic view of an arrangement 1 and an apparatus 2, for analyzing a breathing gas flowing along a breathing tubing 3 such as an endotracheal tube, in which case liquid particles can be delivered at times depending on a phase of a breathing cycle or continuously into the breathing gas for the subject breathing. Components of the apparatus may be part of the arrangement or separate from it. The phases of the breathing cycle are inhalation, exhalation and a time period between these two cycles that are transitions from one to another.

The arrangement may comprise a ventilator 4 connected to the subject 5 for assisting a breathing function of the subject. The ventilator 4 is supplying along a first tubing 6 the volume of the breathing gas for an inspiration and is receiving along a second tubing 7 the volume of the breathing gas for an exhalation. Also the arrangement comprises an airway adapter 8, such as a conventional sidestream type airway adapter, operationally connectable to the breathing tubing 3 for conveying the breathing gas and which adapter is in flow connection with the first tubing 6 and the second tubing 7 and the ventilator 4, typically between the ventilator 4 and the breathing tubing 3 guiding both the breathing gas for the exhalation from lungs of the subject and the breathing gas for the inhalation to the lungs of the subject. The airway adapter is provided with a sample output connector 10 for gas samples supplied along a sampling tube 11 from the breathing gas during at least one of the phases of the breathing cycle, typically during at least one of two phases, which are an inspiration and expiration.

In Figure 1 between the airway adapter 8 and the ventilator 4 there is also a branching unit 9 having at least three limbs, one of them being connected to the first tubing 6, another one being connected to the second tubing 7 and the third one being connected to the airway adapter 8. Additional limbs may be used to connect additional devices to enter breathing circuit and/or subject's lungs. A sampling tube 11 is connected to the sample output connector 10 guiding gas samples to a gas analyzer 12, such as a sidestream type gas analyzer, which is a part of the apparatus 2, placed in Figure 1 outside the ventilator 4, but which could as well be inside the ventilator.

The arrangement in Figure 1 also comprises a liquid supplier 14, such as a nebulizer or a humidifier, for delivering from liquid substance into the breathing gas continuously or at times liquid particles, such as liquid form drug or water particles. The liquid supplier 14 may be connectable into the first tubing 6 between the branching unit 9 and ventilator 4 to generate the liquid particles, which can be as an aerosol in the breathing gas, for example continuously into the first tubing 6 from where the aerosol is further delivered into the subject within the inspiratory gas flow. The generation of liquid particles including the drug may also be intermittent in which case the liquid supplier 14 is turned on and off when needed for example turned on for the time of inspiration only and turned off for the expiration. The liquid particles can also be generated during the period between the inspiration and expiration only or in addition to the inspiration or expiration phase. So this means that the liquid supplier may function intermittently or continuously. Thus the liquid particles can be generated into the first tubing 6 during inspiration and the generation of liquid particles can be turned off during expiration to increase the delivery efficiency and to minimize the amount of wasted drug.

The liquid supplier 14, which is a humidifier for water vapor delivery, can be assembled between the breathing tubing 3 and the airway adapter 4. The active humidifier can evaporate water particles from a separate liquid chamber into the breathing gas, in which case the humidifier can be connectable to any tubing between the ventilator 2 and the subject, but a preferred place is in the branching unit 9 as shown in Figure 1. Also the humidifier could be between the airway adapter 8 and the branching unit 9. The production of liquid particle can be arranged similarly as described hereinbefore.

The arrangement further comprises an indicator 17 for producing a signal indicative of the breathing cycle or the delivery timing of the liquid supplier and for providing a signal through a signal path 15 or wirelessly. The indicator can produce the signal regarding the breathing cycle based on the ventilator information in which case it may also locate in the ventilator, but as well the breathing cycle can be recognized by means of a flow sensor 18 or a pressure sensor 19, which may locate between the branching unit 9 and the breathing tubing 3, indicating the direction of the gas flow, which may prove about an inspiration, an expiration or a period between these two breathing phases. The indicator 17 may also be part of the liquid supplier 14 providing information about the delivery timing of liquid particles into the breathing gas.

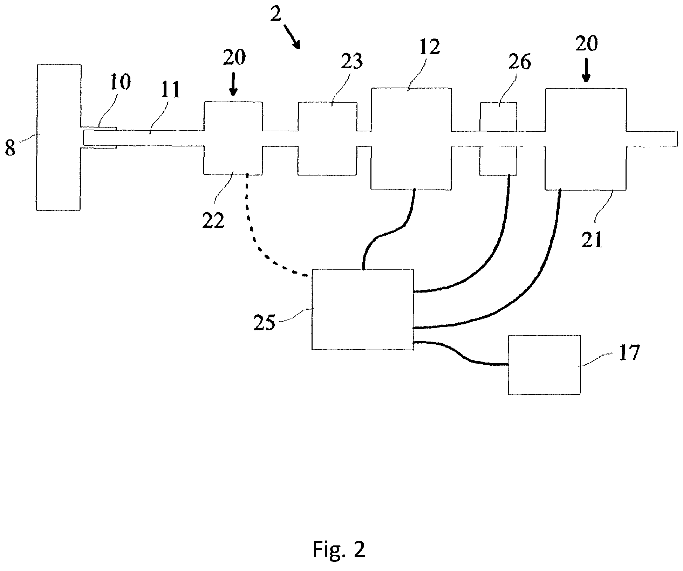

The apparatus comprising the gas analyzer 12 also comprises a gas sample supplier 20, such as a pump 21 or a combination of a valve 22 and a pump 21, to adjust the gas sample supply from the airway adapter 8 along the sampling tube 11 to the gas analyzer 12 as shown in Figure 2 showing some details of the apparatus 2, which is a supplement to Figure 1 with same reference numbers. The gas sample supplier 20, which is a pump 21, typically locates downstream the gas analyzer 12 withdrawing the gas sample through the analyzer to the pump, but in case both the valve 22 and the pump 21 is used as the gas sample supplier, the valve 22 should locate upstream the pump 21, preferably upstream the gas analyzer 12 as shown in Figure 2.

As shown in Figures 1 and 2 the apparatus may also comprise a liquid separator 23, which is upstream the gas analyzer to separate the liquid component following the gas sample withdrawn from the breathing gas flowing through the airway adapter 8. Thus the liquid separator separates the liquid component from the gas component, which is well-known in the art. The liquid separator alone does not solve the problem discussed in this application, because there are liquids or liquid components, which can penetrate the liquid separator and cause harm to the gas analyzer.

To automatically control the gas sample supplier 20 a processing unit 25 as shown in Figure 2 is needed, which may be part of the apparatus. The processing unit 25 can be separate or common with the gas analyzer receiving the signal indicative of gas sample properties, such as one of a respiration rate of a subject and a concentration of at least one component of the breathing gas, from the gas analyzer 12. The processing unit 25 can control the sample gas supplier 20 based on the signal received from the indicator 17 indicative of either the phase of the breathing cycle or the delivery timing of the liquid particles to limit access of liquid particles towards the gas analyzer 12. The gas sample supplier 20 is adapted to decrease the sample flow rate without interrupting the sample flow in case it is advantageous to continuously supply at least a small amount of the sample gas. In that case the supplying rate is adapted to decrease during an inspiration at least 40%, more specifically at least 50% or even more specifically at least 70% compared to the mean supplying rate during the expiration. After the inspiration, when the sample gas supply was decreased or even interrupted, the gas sample supplier is adapted to increase sample flow rate or to restart gas sample supply, if it was interrupted, from the breathing gas to supply gas sample during an exhalation.

By decreasing the sample flow rate during the inspiration, it is supposed that this may decrease sufficiently liquid particles flow to the sampling tube 11 and towards the gas analyzer 12 and thus the negative effect of the liquid particles on the gas analysis can be avoided. However, in most cases it might be advisable to pause the sample gas flow during the inspiration, because then the breathing gas flow towards the subject includes liquid particles of higher concentration and maybe of higher volume than during the expiration. Also usually the samples of the breathing gas supplied during the expiration are more interesting to control of vital functions of the subject than those breathing gas samples acquired during the inspiration. But occasionally also the gas samples of the inspiration may be needed to control the subject.

Also a sample flow sensor 26 as shown in Figure 2, typically upstream the gas sample supplier 20 or the gas analyzer 12, is useful when the sample gas flow rate should be measured. The signal indicative of the sample gas flow rate can be fed to the processing unit 25 to control the sample gas flow rate. It is important that the flow rate of supplied gas is not too high not to harm the subject, by sucking too much breathing gas from the lungs that would collapse the lungs. If the flow rate of supplied gas is too low especially during expiration the gas concentration or respiration rate values would not be accurate. If accurate capnogram is desired the flow rate of supplied gas needs to be measured accurately to restore the correct time line, especially when decreasing the sample flow rate without interrupting the sample flow. When the liquid supply is not present the flow rate of supplied gas is kept constant to get uniform capnogram.

The signal that activates the generation or delivery of liquid particles can be derived along the signal path 15 from the ventilator 4, which signal can be based on the ventilator working cycle or a ventilator flow or pressure. The signal can also be derived along signal path 27 from the flow sensor 18 and/or along a signal path 28 from the pressure sensor 19 connected to a breathing circuit that measures the flow and/or pressure of breathing gas delivered into the subject during inspiration and removed from the patient during expiration. It is also possible that the liquid supplier 14 has measuring device such as a pressure sensor to measure breathing gas pressure and/or a flow sensor to measure flow for turning on and off the liquid particle delivery such as nebulization.

As already explained hereinbefore, when the liquid particle delivery occurs the gas sample supplier 20 may be paused such as turned off for the time of inspiration to prevent liquid particles such as aerosol, which flows within the inspiratory air that comes from the first tubing 6 through the airway adapter 8 past the sample output connector 10, to enter the gas analyzing system. This may also mean that the gas analyzing is paused, because new samples are not received. The liquid supplier 14 or the host device in which the liquid supplier and the gas analyzer 12 are connected to, such as the ventilator 4, can automatically inform the processing unit 25 to turn the gas sample supplier 20 into a functional mode in which the gas analyzer's sample gas flow is paused or readjusted for the time of the liquid particle delivery. Pausing can be based on the same signals as described previously for the liquid supplier 14 or the liquid supplier can provide the signal. It is also possible to switch a bypass valve preferably inside the gas analyzer 12, but also outside the gas analyzer (not shown in Figure 1) to suck zero gas into the gas analyzer, such as filtered room air that can be used for zeroing the gas measurement.

During inspiration liquid particles flow within the inspiratory air through the airway adapter 8 into the subject's lungs, but as the gas sample flow is zero the liquid particles do not flow through the sample output connector 10 into the sampling tube 11 and the gas analyzer 12 keeping it clean and dry of liquid particles. During expiration the sample gas flow is turned on to enable the gas to flow from the breathing circuit through the sample output connector 10 into the sampling tube 11 and the gas analyzer 12 to be analyzed. Whether the liquid supplier 14 produces the liquid particles continuously or if it is turned off for the time of expiration the possible liquid particles coming from the first tubing 6 will more willingly flow away from the subject towards the second tubing 7 and ventilator 4, thus its access into the gas analyzer 12 is substantially prevented. To ensure that the liquid particles in aerosol do not flow into the gas analyzer 12 the liquid supplier that can be turned off for the time of expiration can be used.

Normally, when the liquid particles delivery is not present, the gas analyzer would show continuous capnogram including inspiratory and expiratory values, but now when the liquid particle delivery is present it shows the end tidal (ET) values of breathing gas, which is however the most important information to understand the gas exchange in subjects's lungs for example to control the ventilation of the subject.

It is possible to connect the liquid supplier 14 between the branching unit 9 and airway adapter 8 and also in that case it is possible to prevent liquid particles to enter gas analyzer 12 by pausing or adjusting the sample gas flow for the time of inspiration. During expiration, whether the liquid particle delivery is continuous or intermittently synchronized to inspiration, the expiratory air flowing out from the patient flushes the liquid particles in the breathing air towards the second tubing 7 preventing the aerosol to enter in to the gas analyzer 12.

If the liquid supplier 14 is connected between the airway adapter 4 and the breathing tubing 3 the liquid supplier that generates the liquid particles continuously could not be used especially if the gas analyzing is desired. During inspiration the liquid particles would naturally flow straight into the subject's lungs, but during expiration the liquid particles would flow towards the second tubing 7, through the airway adapter 4 into the gas analyzer 12 as the gas sample supplier sucks gas samples to get ET values for the expiratory gases coming from the patient. The liquid supplier that can be synchronized to inspiration would work well, but in either case as well as that explained hereinbefore, when the liquid supplier is placed between the branching unit 5 and breathing tubing 3, the dead volume that nebulizer adds into the breathing circuit would be high causing rebreathing of gases and could not be used especially with smaller subjects.

Figure 1 also shows an alternative place for the liquid supplier 14 by connecting it into a fourth port 16 of branching unit 9 provided that such port exists in the branching unit. The liquid supplier 14 is positioned close to the intersection of first tubing 6 and second tubing 7 so that the liquid particles delivery is as close to the subject 5 as possible. Since the fourth port 16 is not in the path common for inspiratory and expiratory gases and as there is no air flow through the fourth port, the liquid supplier or the port 16 do not add dead volume into the breathing circuit.

If the liquid supplier 14 generates the liquid particles continuously, during inspiration the liquid particles flow from the port 16 into the subject 1 within the inspiratory air. During expiration the liquid particles flows away from the subject 1 straight from the first tubing 6 into the second tubing 7 and towards the ventilator 4. Samples of breathing gases can be analyzed by pausing the sample gas flow for the time of inspiration and starting the sample gas flow again for the time of exhalation. Even to minimize the flow of liquid particles into the gas analyzer the sample gas flow can be turned on after a predetermined time delay in regard to start of exhalation. The length of the time delay is typically short, but proportional to the time that is needed to empty the dead volume of breathing gas including liquid particles before the used gas coming from patient's lungs flushes the dead volume of air with liquid particles.

If the liquid supplier 14 can be turned on to generate liquid particles for the time of inspiration and turned off to stop liquid particles generation for the time of expiration the delivery efficiency of liquid particles can be increased as more liquid particles flows into the patient's lungs and less liquid particles is wasted into the breathing circuit and devices connected to that. Breathing gases can be analyzed by pausing the sample gas flow for the time of inspiration and restarting the sample gas flow again for the time of expiration. To minimize the flow of liquid particles into the gas analyzer the sample gas flow can be turned on after a predetermined time delay in regard to start of exhalation. The length of time delay is typically short, but proportional to the time that is needed to empty the dead volume of air comprising liquid particles before the used gas coming from patient's lungs flushes the dead volume of air with liquid particles. Another way to minimize the flow of liquid particles into the gas analyzer is to stop the liquid particles delivery shortly before the end of inspiration to minimize the delivery of liquid particles into the dead volume of the breathing circuit. When the exhalation starts the dead volume is already empty of liquid particles and the sample gas flow can be turned on just at the beginning of exhalation.

If inspired gases need to be analyzed during liquid particles delivery and if the liquid supplier is available, which liquid particle generation can be turned on and off freely, it is possible to stop or skip liquid particle delivery during every second, third, fourth etc. inspirations. During these inspirations, when the liquid particle delivery is stopped, the gas sample supplier 13 can suck gas samples from the breathing circuit to be analyzed by the gas analyzer 12. It is possible as inspired gas measurement values are used less frequently than expired values, but gas analyzing during some inspiration would of course increase the time of the liquid particle delivery depending on how frequently it is executed.

The written description uses examples to disclose the invention, including the best mode, and also to enable any person skilled in the art to make and use the invention. The patentable scope of the invention is defmed by the claims.

## Claims

1. An apparatus for analyzing a breathing gas flowing along a breathing tubing for subject breathing, the breathing gas comprising breathing cycles having different phases, liquid particles adapted to be delivered at times depending on the phase of the breathing cycle or continuously into the breathing gas, the apparatus comprising:
a gas sample supplier (20) adapted to adjust gas sample supply from the breathing gas; and
a gas analyzer (12) adapted to receive the gas sample adjusted by said gas sample supplier (20) and adapted to measure a gas sample property of the breathing gas;
an indicator (17) for producing a signal indicative of the breathing cycle or the delivery timing of the liquid supplier;
the apparatus also comprising a processing unit (25) adapted to receive a signal indicative of one of the phase of the breathing cycle and the delivery timing of the liquid particles from the indicator, said processing unit (25) being adapted to control said gas sample supplier (20) based on the signal received by said processing unit (25) to limit access of liquid particles with the gas sample towards said gas analyzer (12); and
**characterized in that** said gas sample supplier (20) is adapted to decrease sample flow rate under control of said processing unit (25) during an inspiration phase compared to the mean supplying rate during the expiration phase to limit access of liquid particles with the gas sample towards said gas analyzer (12).

2. The apparatus according to claim 1, further comprising a liquid separator (23) upstream said gas analyzer (12) adapted to separate a liquid component from said gas sample, said gas analyzer (12) being adapted to measure the gas sample property, which is one of a respiration rate of a subject and a concentration of at least one component of the breathing gas.

3. The apparatus according to claim 1, further comprising a sample flow sensor (26) adapted to indicate the sample gas flow rate.

4. The apparatus according to claim 1, **characterized in that** said gas sample supplier (20) is adapted to decrease sample flow rate or pause sample flow towards said gas analyzer (12) under control of said processing unit (25) when one of the concentration and the volume of the liquid particles in the sample flow is increasing and that said gas sample supplier (20) is adapted to increase sample flow rate or restart sample flow when one of the concentration and the volume of liquid particles in the sample flow is decreasing.

5. The apparatus according to claim 1, **characterized in that** said gas sample supplier is a pump (21) adapted to withdraw gas sample from the breathing gas, or that said gas sample supplier comprises a valve (22) and a pump (21), which valve is upstream one of said gas analyzer (12) and said pump, said valve (22) being adapted to limit access of liquid particles with the gas sample withdrawn by said pump towards said gas analyzer (12).

6. The apparatus according to claim 1, **characterized in that** said processing unit (25) is adapted to control said gas sample supplier (20) based on the signal indicative of the phase of the breathing cycle, which is one of an inspiration, expiration and time period between the inspiration and expiration.

7. The apparatus according to claim 1, **characterized in that** said gas sample supplier (20) is adapted to decrease sample flow rate at least 40%, more specifically at least 50% or even more specifically at least 70% during inspiration compared to the mean supplying rate during an expiration without pausing sample flow during an inspiration.

8. The apparatus according to claim 1, **characterized in that** said gas sample supplier (20) controlled by said processing unit (25) is adapted to decrease sample flow rate or even to pause gas sample supply for an inspiration to limit access of liquid particles and to increase sample flow rate or to restart gas sample supply from the breathing gas to supply gas sample during an expiration.

9. The apparatus according to claim 8, **characterized in that** said gas sample supplier (20) controlled by said processing unit (25), which is adapted to increase sample flow rate or to restart gas sample supply from the breathing gas to supply gas sample during expiration, is adapted to do that after a predetermined time delay in regards to the start of expiration.

10. The apparatus according to claim 1, further comprising an airway adapter (8) operationally connectable to said breathing tubing for conveying the breathing gas and which adapter is provided with a sample output connector (10) for gas samples adapted to be supplied along a sampling tube (11) from the breathing gas during at least one of the phase of the breathing cycle.

11. The apparatus according to claim 10, further comprising a ventilator (4) operationally connectable to said airway adapter (8) adapted to assist breathing function of the subject..

12. The apparatus according to claim 1, further comprising a liquid supplier (14) adapted to deliver from liquid substance continuously or at times liquid particles into the breathing gas and that said liquid supplier is one of a nebulizer to deliver liquid form drugs into the breathing gas and a humidifier to deliver water vapor into the breathing gas.

13. The apparatus according to claim 12, further comprising an indicator (17) adapted to provide a signal indicative of one of the phase of the breathing cycle and the delivery timing of said liquid supplier (14).

14. The apparatus according to any preceding claim, wherein the gas sample supplier (20) is adapted to decrease the sample flow rate without interrupting the sample flow.

## Patentansprüche

1. Vorrichtung zum Analysieren eines Atemgases, das eine Röhrenleitung entlang zur Personenbeatmung strömt, wobei das Atemgas Atmungszyklen mit verschiedenen Phasen umfasst, wobei flüssige Partikel dazu geeignet sind, abhängig vom Atmungszyklus bisweilen oder fortlaufend in das Atemgas zugeführt zu werden, die Vorrichtung umfassend:
einen Gasprobenzuführer (20), der zum Anpassen von Gasprobenzuführung aus dem Atemgas geeignet ist; und
einen Gasanalysator (12), der zum Aufnehmen der Gasprobe, die durch den Gasprobenzuführer (20) angepasst wurde, geeignet ist und zum Messen einer Gasprobeneigenschaft des Atemgases geeignet ist;
einen Indikator (17) zum Erzeugen eines Signals, das den Atmungszyklus oder die Zufuhrzeiteinstellung des Flüssigkeitszuführers anzeigt;
wobei die Vorrichtung außerdem eine Verarbeitungseinheit (25) umfasst, die zum Empfangen eines Signals geeignet ist, das eines der Phase des Atmungszyklus und der Zufuhrzeiteinstellung der flüssigen Partikel vom Indikator geeignet ist, wobei die Verarbeitungseinheit (25) zum Steuern des Gasprobenzuführers (20) auf Grundlage des Signals, das durch die Verarbeitungseinheit (25) empfangen wurde, geeignet ist, um Zugang von flüssigen Partikeln mit der Gasprobe zum Gasanalysator (12) hin zu begrenzen; und
**dadurch gekennzeichnet, dass** der Gasprobenzuführer (20) zum Herabsetzen der Probenströmungsrate unter Steuerung der Verarbeitungseinheit (25) während einer Einatmungsphase im Vergleich zur mittleren Zufuhrrate während der Ausatmungsphase geeignet ist, um Zugang von flüssigen Partikeln mit der Gasprobe zum Gasanalysator (12) hin zu begrenzen.

2. Vorrichtung nach Anspruch 1, ferner umfassend einen Flüssigkeitsabscheider (23), der dem Gasanalysator (12) vorgelagert ist und zum Abscheiden einer flüssigen Komponente aus der Gasprobe geeignet ist, wobei der Gasanalysator (12) zum Messen der Gasprobeneigenschaft geeignet ist, die eine einer Atmungsrate einer Person und einer Konzentration von mindestens einer Komponente des Atemgases ist.

3. Vorrichtung nach Anspruch 1, ferner umfassend einen Probenströmungssensor (26), der zum Anzeigen der Probengasströmungsrate geeignet ist.

4. Vorrichtung nach Anspruch 1, **dadurch gekennzeichnet, dass** der Gasprobenzuführer (20) zum Herabsetzen der Probenströmungsrate oder Pausieren der Probenströmung zum Gasanalysator (12) hin unter Steuerung der Verarbeitungseinheit (25) geeignet ist, wenn eines der Konzentration und des Volumens der flüssigen Partikel in der Probenströmung zunimmt, und dass der Gasprobenzuführer (20) zum Erhöhen der Probenströmungsrate oder Neustarten der Probenströmung geeignet ist, wenn eines der Konzentration und des Volumens von flüssigen Partikel in der Probenströmung abnimmt.

5. Vorrichtung nach Anspruch 1, **dadurch gekennzeichnet, dass** der Gasprobenzuführer eine Pumpe (21) ist, die zum Entziehen einer Gasprobe aus dem Atemgas geeignet ist, oder dass der Gasprobenzuführer ein Ventil (22) und eine Pumpe (21) umfasst, wobei das Ventil einem des Gasanalysators (12) und der Pumpe vorgelagert ist, wobei das Ventil (22) zum Begrenzen des Zugangs von flüssigen Partikeln mit der Gasprobe, die durch die Pumpe entzogen wurde, zum Gasanalysator (12) hin geeignet ist.

6. Vorrichtung nach Anspruch 1, **dadurch gekennzeichnet, dass** die Verarbeitungseinheit (25) zum Steuern des Gasprobenzuführers (20) auf Grundlage des Signals, das die Phase des Atmungszyklus anzeigt, geeignet ist, der eines eines Einatmens, Ausatmens oder einer Zeitperiode zwischen dem Einatmen und Ausatmen ist.

7. Vorrichtung nach Anspruch 1, **dadurch gekennzeichnet, dass** der Gasprobenzuführer (20) zum Herabsetzen der Probenströmungsrate um mindestens 40%, insbesondere mindestens 50% oder sogar weitergehend insbesondere mindestens 70% während des Einatmens im Vergleich zur mittleren Zufuhrrate während eines Ausatmens ohne Pausieren der Probenströmung während eines Einatmens geeignet ist.

8. Vorrichtung nach Anspruch 1, **dadurch gekennzeichnet, dass** der Gasprobenzuführer (20), der durch die Verarbeitungseinheit (25) gesteuert wird, zum Herabsetzen der Probenströmungsrate oder sogar zum Pausieren der Gasprobenzufuhr für ein Einatmen zum Begrenzen des Zugangs von flüssigen Partikeln und zum Erhöhen der Probenströmungsrate oder zum Neustarten der Gasprobenzufuhr aus dem Atmungsgas zum Zuführen einer Gasprobe während eines Ausatmens geeignet ist.

9. Vorrichtung nach Anspruch 8, **dadurch gekennzeichnet, dass** der Gasprobenzuführer (20), der durch die Verarbeitungseinheit (25) gesteuert wird und zum Erhöhen der Probenströmungsrate oder zum Neustarten der Gasprobenzufuhr aus dem Atemgas zum Zuführen einer Gasprobe während des Ausatmens geeignet ist, dazu geeignet ist, dies nach einer vorbestimmten Zeitverzögerung hinsichtlich des Ausatmungsbeginns auszuführen.

10. Vorrichtung nach Anspruch 1, ferner umfassend einen Luftwegadapter (8), der zum Befördern des Atemgases betriebsfähig mit der Atmungsröhrenleitung verbindbar ist, und wobei der Adapter mit einem Probenausgangsverbinder (10) für Gasproben versehen ist, die dazu geeignet sind, entlang einer Probenerfassungsröhre (11) aus dem Atemgas während mindestens einer der Phase des Atmungszyklus zugeführt zu werden.

11. Vorrichtung nach Anspruch 10, ferner umfassend ein Gebläse (4), das betriebsfähig mit dem Luftwegadapter (8) verbindbar ist und zum Unterstützen der Atmungsfunktion der Person geeignet ist.

12. Vorrichtung nach Anspruch 1, ferner umfassend einen Flüssigkeitszuführer (14), der zum fortlaufenden oder bisweiligen Zuführen von flüssigen Partikeln aus Flüssigkeitssubstanz in das Atemgas geeignet ist, und wobei der Flüssigkeitszuführer einer von einem Zerstäuber zum Zuführen von Medikamenten in flüssiger Form in das Atemgas und einem Befeuchter zum Zuführen von Wasserdampf in das Atemgas ist.

13. Vorrichtung nach Anspruch 12, ferner umfassend einen Indikator (17), der zum Vorsehen eines Signals geeignet ist, das eines der Phase des Atmungszyklus und der Zufuhrzeiteinstellung des Flüssigkeitszuführers (14) anzeigt.

14. Vorrichtung nach einem der vorstehenden Ansprüche, wobei der Gasprobenzuführer (20) dazu geeignet ist, die Probenströmungsrate herabzusetzen, ohne die Probenströmung zu unterbrechen.

## Revendications

1. Appareil d'analyse d'un gaz respiratoire s'écoulant le long d'une tubulure de respiration pour la respiration d'un sujet, le gaz respiratoire comprenant des cycles respiratoires ayant différentes phases, des particules liquides adaptées pour être distribuées à certains moments en fonction de la phase du cycle respiratoire ou continuellement dans le gaz respiratoire, l'appareil comprenant :
un dispositif d'alimentation en échantillon de gaz (20) adapté pour ajuster l'alimentation en échantillon de gaz à partir du gaz respiratoire ; et
un analyseur de gaz (12) adapté pour recevoir l'échantillon de gaz ajusté par ledit dispositif d'alimentation en échantillon de gaz (20) et adapté pour mesurer une propriété de l'échantillon de gaz du gaz respiratoire ;
un indicateur (17) pour produire un signal indicateur du cycle respiratoire ou de la programmation de la distribution du dispositif d'alimentation en liquide ;
l'appareil comprenant également une unité de traitement (25) adaptée pour recevoir un signal indicateur de l'une de la phase du cycle respiratoire et de la programmation de la distribution des particules liquides à partir de l'indicateur, ladite unité de traitement (25) étant adaptée pour commander ledit dispositif d'alimentation en échantillon de gaz (20) sur la base du signal reçu par ladite unité de traitement (25) pour limiter l'accès des particules liquides avec l'échantillon de gaz vers ledit analyseur de gaz (12) ; et
**caractérisé en ce que** ledit dispositif d'alimentation en échantillon de gaz (20) est adapté pour diminuer le débit de l'échantillon sous le contrôle de ladite unité de traitement (25) durant une phase d'inspiration comparativement au taux d'alimentation moyen durant la phase d'expiration pour limiter l'accès des particules liquides avec l'échantillon de gaz vers ledit analyseur de gaz (12).

2. Appareil selon la revendication 1, comprenant en outre un séparateur de liquide (23) en amont dudit analyseur de gaz (12) adapté pour séparer un composant liquide dudit échantillon de gaz, ledit analyseur de gaz (12) étant adapté pour mesurer la propriété de l'échantillon de gaz, qui est l'une d'une fréquence respiratoire d'un sujet et d'une concentration d'au moins un composant du gaz respiratoire.

3. Appareil selon la revendication 1, comprenant en outre un capteur de débit d'échantillon (26) adapté pour indiquer le débit d'un échantillon de gaz.

4. Appareil selon la revendication 1, **caractérisé en ce que** ledit dispositif d'alimentation en échantillon de gaz (20) est adapté pour diminuer le débit de l'échantillon ou suspendre l'écoulement de l'échantillon vers ledit analyseur de gaz (12) sous le contrôle de ladite unité de traitement (25) lorsque l'un de la concentration et du volume des particules liquides dans l'écoulement de l'échantillon augmente et que ledit dispositif d'alimentation en échantillon de gaz (20) est adapté pour augmenter le débit de l'échantillon ou redémarrer l'écoulement de l'échantillon lorsque l'un de la concentration et du volume de particules liquides dans l'écoulement de l'échantillon diminue.

5. Appareil selon la revendication 1, **caractérisé en ce que** ledit dispositif d'alimentation en échantillon de gaz est une pompe (21) adaptée pour prélever un échantillon de gaz à partir du gaz respiratoire, ou **en ce que** ledit dispositif d'alimentation en échantillon de gaz comprend une valve (22) et une pompe (21), laquelle valve est en amont de l'un dudit analyseur de gaz (12) et de ladite pompe, ladite valve (22) étant adaptée pour limiter l'accès des particules liquides avec l'échantillon de gaz prélevé par ladite pompe vers ledit analyseur de gaz (12).

6. Appareil selon la revendication 1, **caractérisé en ce que** ladite unité de traitement (25) est adaptée pour commander ledit dispositif d'alimentation en échantillon de gaz (20) sur la base du signal indicateur de la phase du cycle respiratoire, qui est l'une d'une inspiration, d'une expiration et d'un délai entre l'inspiration et l'expiration.

7. Appareil selon la revendication 1, **caractérisé en ce que** ledit dispositif d'alimentation en échantillon de gaz (20) est adapté pour diminuer le débit de l'échantillon d'au moins 40%, plus spécifiquement d'au moins 50% ou encore plus spécifiquement d'au moins 70% durant l'inspiration comparativement au taux d'alimentation moyen durant une expiration sans suspension de l'écoulement de l'échantillon durant une inspiration.

8. Appareil selon la revendication 1, **caractérisé en ce que** ledit dispositif d'alimentation en échantillon de gaz (20) commandé par ladite unité de traitement (25) est adapté pour diminuer le débit de l'échantillon ou même suspendre l'alimentation en échantillon de gaz pour une inspiration pour limiter l'accès des particules liquides et pour augmenter le débit d'échantillon ou pour redémarrer l'alimentation en échantillon de gaz à partir du gaz respiratoire pour alimenter l'échantillon de gaz durant une expiration.

9. Appareil selon la revendication 8, **caractérisé en ce que** ledit dispositif d'alimentation en échantillon de gaz (20) commandé par ladite unité de traitement (25), qui est adapté pour augmenter le débit d'échantillon ou pour redémarrer l'alimentation en échantillon de gaz à partir du gaz respiratoire pour alimenter l'échantillon de gaz durant l'expiration, est adapté pour faire ceci après un temps de retard prédéterminé au regard du début de l'expiration.

10. Appareil selon la revendication 1, comprenant en outre un adaptateur pour voies aériennes (8) pouvant être raccordé de manière opérationnelle à ladite tubulure de respiration pour transporter le gaz respiratoire et lequel adaptateur est doté d'un connecteur de sortie d'échantillon (10) pour échantillons de gaz adapté pour être fourni le long d'un tube d'échantillonnage (11) à partir du gaz respiratoire durant au moins l'une de la phase du cycle respiratoire.

11. Appareil selon la revendication 10, comprenant en outre un insufflateur (4) pouvant être raccordé de manière opérationnelle audit adaptateur pour voies aériennes (8) adapté pour assister la fonction respiratoire du sujet.

12. Appareil selon la revendication 1, comprenant en outre un dispositif d'alimentation en liquide (14) adapté pour distribuer à partir d'une substance liquide continuellement ou à certains moments des particules liquides dans le gaz respiratoire et ce dit dispositif d'alimentation en liquide est l'un d'un nébuliseur pour distribuer des médicaments sous forme liquide dans le gaz respiratoire et d'un humidificateur pour distribuer de la vapeur d'eau dans le gaz respiratoire.

13. Appareil selon la revendication 12, comprenant en outre un indicateur (17) adapté pour fournir un signal indicateur de l'une de la phase du cycle respiratoire et de la programmation de la distribution dudit dispositif d'alimentation en liquide (14).

14. Appareil selon une quelconque revendication précédente, dans lequel le dispositif d'alimentation en échantillon de gaz (20) est adapté pour diminuer le débit d'échantillon sans interruption de l'écoulement de l'échantillon.
